(19) Europäisches Patentamt
European Patent Office
Office européen des brevets

(11) Numéro de publication: 0 092 479
B1

(12) # FASCICULE DE BREVET EUROPEEN

(45) Date de publication du fascicule du brevet: **29.01.86**

(51) Int. Cl.⁴: **C 07 D 251/70**, C 07 D 405/12, C 07 D 409/12, A 61 K 31/53

(21) Numéro de dépôt: **83400755.1**

(22) Date de dépôt: **18.04.83**

(54) **Polymethylène diamines, n,n'-substituées, leurs procédés de préparation et les compositions pharmaceutiques les renfermant.**

(30) Priorité: **21.04.82 FR 8206813**

(43) Date de publication de la demande: **26.10.83 Bulletin 83/43**

(45) Mention de la délivrance du brevet: **29.01.86 Bulletin 86/5**

(84) Etats contractants désignés: **AT BE CH DE FR GB IT LI LU NL SE**

(56) Documents cités:
**FR - A - 1 451 232**
**FR - A - 1 507 062**
**FR - A - 2 019 646**
**FR - A - 2 130 184**
**GB - A - 1 017 862**

(73) Titulaire: **ADIR, 22, rue Garnier, F-92200 Neuilly-sur-Seine (FR)**

(72) Inventeur: **Regnier, Gilbert, Avenue du Plessis, F-92200 Châtenay-Malabry (FR)**
Inventeur: **Dhainaut, Alain, 7 rue des Guipières, F-78400 Chatou (FR)**
Inventeur: **Laubie, Michel, 35 avenue Foch, F-92420 Vaucresson (FR)**
Inventeur: **Duhault, Jacques, 14 bis rue Paul Demange, F-78290 Croissy-sur-Seine (FR)**

(74) Mandataire: **Reverbori, Marcelle et al, ADIR 22 Rue Garnier, F-92200 Neuilly-sur-Seine (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention a pour objet de nouvelles polyméthylène diamines N,N'-substituées, leurs procédés de préparation et les compositions pharmaceutiques les renfermant.

Elle concerne particulièrement les polyméthylène diamines N,N'-substituées de formule générale I:

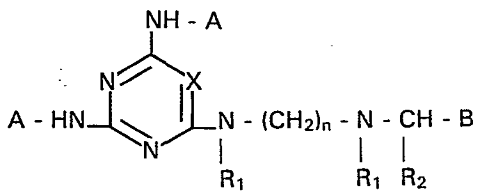

(I)

dans laquelle:

A représente un radical hydro-carboné ayant de 3 à 5 atomes de carbone en chaîne droite ou ramifiée, renfermant éventuellement une ou deux double liaisons, et éventuellement substitué par un ou plusieurs radicaux hydroxyles;

X représente le groupe -CH- ou un atome d'azote;

n représente un nombre entier de 2 à 6;

$R_1$ représente un atome d'hydrogène ou un radical alcoyle ayant de 1 à 5 atomes de carbone;

$R_2$ représente un atome d'hydrogène, un radical alcoyle ayant de 1 à 5 atomes de carbone, un radical cycloalcoyle ayant de 5 à 7 atomes de carbone, ou un radical phényle éventuellement substitué par un ou plusieurs atomes de fluor ou de chlore: et

B représente:

un radical phényle, naphtyle, benzofurannyle, benzothiényle, benzodioxolyle, benzodioxannyle, benzodioxinnyle, Δ3-chroményle, thiochroményle, ou chromannyle, chacun éventuellement substitué par un ou plusieurs atomes de fluor ou de chlore ou radicaux alcoyle ou alcoxy ayant chacun de 1 à 5 atomes de carbone, ou méthylènedioxy, ou

un radical de formule 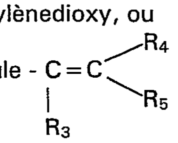

dans laquelle:

$R_3$, $R_4$ et $R_5$ identiques ou différents, représentent chacun un atome d'hydrogène ou un radical phényle éventuellement substitué par un ou plusieurs atomes de chlore ou de fluor.

La présente invention a également pour objet le procédé de préparation des dérivés de formule générale I, caractérisé en ce que l'on condense une diamine de formule générale II:

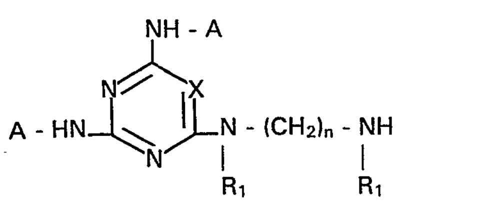

(II)

dans laquelle A, X, n et $R_1$ ont les definitions énoncées précédemment,

avec un dérivé halogéné de formule générale III:

Hal - CH - B (III)
|
$R_2$

dans laquelle $R_2$ et B ont les significations précédemment définies et Hal représente un atome d'halogène tel que par exemple un atome de chlore ou de brome.

La condensation s'effectue de préférence dans un solvant choisi parmi les hydrocarbures benzéniques à haut point d'ébullition comme le toluène ou le xylène, les amides aliphatiques comme le diméthylformamide ou le diméthylacétamide éventuellement mélangés à un hydrocarbure benzénique à haut point d'ébullition, ou le cyanure de méthyle. Il est avantageux d'opérer à une température comprise entre 120 et 140°C en présence d'un accepteur de l'hydracide formé au cours de la réaction. Cet accepteur peut être choisi parmi les carbonates alcalins comme le carbonate de potassium, la triéthylamine ou un excès de la diamine de formule II utilisée pour la condensation.

La présente invention a aussi pour objet le procédé de préparation des dérivés de formule générale I, caractérisé en ce que l'on condense:

un dérivé halogéné de formule générale IV:

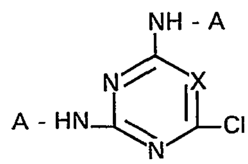

(IV)

dans laquelle A et X ont les significations précédemment définies avec une diamine de formule générale V:

HN - $(CH_2)_n$ - N - CH - B (V)
| | |
$R_1$ $R_1$ $R_2$

dans laquelle $R_1$, $R_2$, n et B ont les significations énoncées précédemment.

La condensation s'effectue de façon particulièrement adéquate dans un solvant choisi parmi les alcools en $C_4$ ou $C_5$ tels que le butanol ou le pentanol, et les amides aliphatiques comme le diméthylformamide ou le diméthylacétamide. Il est recommandé d'opérer à une température comprise entre 120 et 150°C en présence d'un accepteur de l'hydracide formé au cours de la réaction. Cet accepteur peut être choisi parmi les carbonates alcalins comme le carbonate de potassium, la triéthylamine ou un excès de l'amine de formule générale V précédemment définie.

La présente invention a aussi pour objet le procédé de préparation des dérivés de formule générale I':

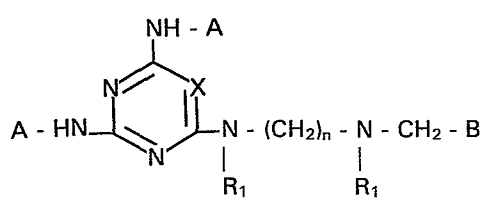

(I')

dans laquelle A, X, n, $R_1$ et B sont tels que précédemment définis, caractérisé en ce que l'on réduit l'amide correspondante de formule générale VI:

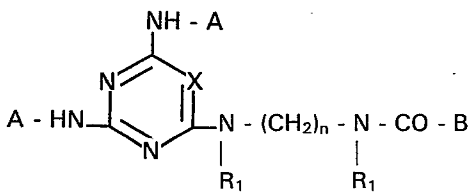
(VI)

dans laquelle A, X, n, $R_1$ et B sont tels que définis précédemment.

Comme agent réducteur on peut utiliser par exemple l'hydrure double de lithium aluminium: Li Al $H_4$ ou l'hydrure de bore: $B_2H_6$.

Une mise en oeuvre particulièrement adéquate consiste à effectuer la réduction dans un solvant comme le tétrahydrofuranne à une température comprise entre 20 et 60°C.

Ces nouveaux dérivés (I) ainsi obtenus peuvent être transformés en sels d'addition avec les acides, sels qui font, à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthane sulfonique et iséthionique.

Ces nouveaux dérivés peuvent être purifiés par des méthodes physiques telles que cristallisation, chromatographie ou chimiques telles que formation de sels d'addition avec des acides et décomposition de ces sels par des agents alcalins.

Les matières premières utilisées dans les procédés précédemment décrits sont soit des produits connus, soit des produits préparés à partir de substances connues, selon des procédés décrits pour préparer des produits analogues comme indiqué dans les exemples suivants.

Les dérivés de formule générale I et leurs sels d'addition physiologiquement tolérables possèdent des propriétés pharmacologiques et thérapeutiques intéressantes. En particulier, ils favorisent la captation d'oxygène et permettent ainsi leur utilisation comme médicament, notamment dans le traitement de tout type d'hypoxie tissulaire.

Ces dérivés et leurs sels physiologiquement tolérables présentent de plus une très faible toxicité.

L'effet des dérivés de l'invention sur la pression d'oxygène (P $O_2$) a été étudié chez le chien anesthésié au Nembutal. Des échantillons de sang sont prélevés périodiquement 2, 5, 15, 45 et 75 minutes après l'administration des composés à tester; ils servent à la détermination du pH, de la P $O_2$ et de la P $CO_2$.

La P $O_2$ est mesurée sur un appareil Radiometer $BMS_3$. La lecture de la P $O_2$ se fait sur cet appareil préalablement étalonné avec des valeurs connues, à l'aide d'une électrode de platine, ou électrode de Clark.

Les produits ont été administrés chez le chien par la voie intraveineuse à la dose de 1 mg/kg et la détermination du pourcentage d'augmentation de la teneur en oxygène du sang artériel montre que ce pourcentage peut selon les composés atteindre jusqu'à 37%, 45 minutes après l'administration du composé, et jusqu'à 43%, 75 minutes après l'administration du composé.

Les produits de la présente invention se sont révélés également actifs dans le traitement de l'hypoxie anémique induite par voie chimique par injection sous-cutanée de $NaNO_2$ selon la méthode de Gibson G.E. Neurobiol Aging *2,* 165, (1981) et Biochem. Pharmacol. *28,* 747, (1979), et dans l'hypoxie hypobare selon la technique de Legeai J.M. et coll. Experientia, *37,* 292, (1981).

La présente invention a également pour objet les compositions pharmaceutiques contenant comme principe actif un dérivé de formule générale I ou un de ses sels physiologiquement tolérables, mélangés ou associés à un excipient pharmaceutique approprié.

Elle concerne notamment les formes dosées renfermant de 20 à 100 mg de principe actif.

Les compositions pharmaceutiques ainsi obtenues sont présentées avantageusement sous des formes dosées diverses telles que par exemple, comprimés, dragées, gélules, suppositoires, solutions injectables ou buvables. Elles peuvent être administrées par la voie orale, rectale ou parentérale aux doses de 20 à 100 mg 1 à 2 fois par jour.

Les exemples suivants donnés à titre non limitatif illustrent l'invention.

*Exemple 1*

N-éthyl N-(bis allylamino-4,6 s.triazinyl-2) N'-éthyl N'-(bis p.fluorobenzhydryl) éthylènediamine

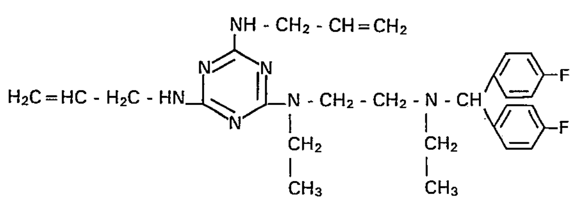

*1ère méthode:*

On chauffe pendant 8 heures à reflux une solution de 6,1 g de N-(bis allylamino-4,6 s.triazinyl-2) N,N'-diéthyl éthylène diamine et de 5,7 g de bromure de bis p.fluorobenzhydryle dans 100 ml de toluène contenant 5% de diméthylformamide, en présence de 2 g de triéthylamine. Ensuite, on refroidit, reprend par 100 ml d'eau, décante et extrait avec 2 fois 50 ml de solution normale d'acide monométhane sulfonique. La solution acide est ensuite lavée à l'éther puis alcalinisée à pH 9 avec $K_2CO_3$. On extrait au chloroforme plusieurs fois. Après évaporation du chloroforme on recueille une huile brute qu'on cristallise dans 160 ml d'éthanol. On isole finalement 6,8 g de N-éthyl N-(bis allylamino-4,6 s.triazinyl-2) N'-éthyl N'-(bis p.fluorobenzhydryl) éthylène diamine, sous forme de cristaux fondant (capillaire) à 105-107°C. L'amine de départ, dont le difumarate fond (Kofler) à 204°C a été préparée par chauffage de la bis allylamino-2,4 chloro-6 s.triazine dans un excès de N,N'-diéthyl éthylène diamine à 140°C.

*2ème méthode:*

On chauffe pendant 5 heures à reflux une solution de 4,25 g de bis allylamino-4,6 chloro-2 s-triazine et de 6 g de N,N'-diéthyl N-(bis p.fluorobenzhydryl) éthylène diamine dans 50 ml de butanol, en présence de 100 mg d'iodure de sodium et 2,6 ml de triéthylamine. On chasse ensuite le solvant sous pression réduite. Le résidu est recristallisé dans 160 ml d'éthanol à 70%. On obtient 5,2 g de N-éthyl N-(bis allylamino-4,6 s-triazinyl-2) N'-éthyl N'-(bis p.fluorobenzhydryl) éthylène diamine, sous forme de cristaux fondant (capillaire) à 105-107°C. L'amine de départ (Eb/27 Pa = 154-156°C) a été préparée par chauffage du bromure de bis p.fluorobenzhydryle dans un excès de N,N'-diéthyl éthylène diamine.

*Exemples 2 à 14*

Les dérivés suivants ont été préparés selon les méthodes décrites dans l'exemple 1:

2) N-(bis allylamino-4,6 s.triazinyl-2) N'-(bis p.-fluorobenzhydryl) éthylène diamine, P.F. (capillaire) du difumarate correspondant: 185-187°C (éthanol).

3) N-méthyl N-(bis allylamino-4,6 s.triazinyl-2) N'-méthyl N'-(bis p.fluorobenzhydryl) éthylène diamine, P.F. (capillaire) du fumarate correspondant: 139-142°C (éthanol).

4) N-(bis allylamino-4,6 s.triazinyl-2) N'-(bis p.-fluorobenzhydryl) triméthylène diamine, P.F. (capillaire) du difumarate correspondant: 177-179°C (éthanol).

5) N-(bis allylamino-4,6 s.triazinyl-2) N'-(α-cyclopentyl p.fluorobenzyl) éthylène diamine, P.F. (Kofler) du fumarate correspondant: 117°C (éthanol).

6) N-(bis allylamino-4,6 s.triazinyl-2) N-(α-cyclohexyl p.fluorobenzyl) éthylène diamine, P.F. (Kofler) du difumarate correspondant: 204°C (éthanol).

7) N-éthyl N-(bis allylamino-4,6 s.triazinyl-2) N'-éthyl N'-(p.fluorobenzyl) éthylène diamine, P.F. (Kofler) du trifumarate correspondant: 154°C (éthanol anhydre).

8) N-éthyl N-(bis allylamino-4,6 s.triazinyl-2) N'-éthyl N'-(benzofurannyl-2 méthyl) éthylène diamine, P.F. (Kofler) du difumarate correspondant: 162°C (éthanol).

9) N-éthyl N-(bis allylamino-4,6 s.traizinyl-2) N'-éthyl N'-(benzothiényl-2 méthyl) éthylène diamine, P.F. (Kofler) du fumarate correspondant: 148°C (éthanol).

10) N-éthyl N-(bis allylamino-4,6 s.triazinyl-2) N'-éthyl N'-(bis p.fluorobenzhydryl) triméthylène diamine, P.F. (Kofler) du difumarate correspondant: 125°C (éthanol anhydre).

11) N-éthyl N-(bis allylamino-4,6 s.triazinyl-2) N'-éthyl N'-cinnamyl éthylène diamine, P.F. (Kofler) du dioxalate correspondant: 171-173°C.

12) N-éthyl N-(bis allylamino-4,6 s.triazinyl-2) N'-éthyl N'-(bis p.fluorophényl-3,3 allyl) éthylène diamine.

13) N-éthyl N-(bis allylamino-4,6 s.triazinyl-2) N'-éthyl N'-(benzothiényl-2 méthyl) triméthylène diamine, P.F. (Kofler) du difumarate correspondant: 130°C (éthanol anhydre).

14) N-éthyl N-(bis allylamino-4,6 s.triazinyl-2) N'-éthyl N'-cinnamyl triméthylène diamine, P.F. (capillaire) du difumarate correspondant: 114-116°C (éthanol anhydre).

*Exemple 15*

N-éthyl N-(bis allylamino-4,6 s.triazinyl-2) N'-éthyl N'-(benzothiényl-2 méthyl) éthylène diamine:

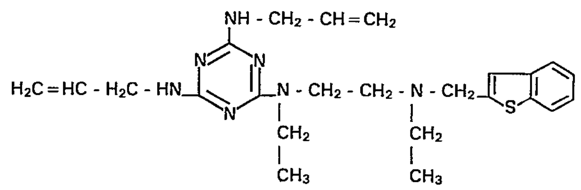

On chauffe pendant 18 heures à reflux une solution de 9,3 g de N-éthyl N-(bis allylamino-4,6 s.triazinyl-2) N'-éthyl N'-(benzothiényl-2 carbonyl) éthylène diamine, P.F. (Kofler) du fumarate correspondant: 180°C, dans 200 ml de tétrahydrofuranne en présence de 2,28 g de Li Al $H_4$. Au bout de ce temps, on refroidit sous azote et hydrolyse le complexe avec successivement 2,28 ml d'eau, 2,28 ml de solution 4N de soude et 7 ml d'eau. On filtre l'alumine formée et évapore le filtrat à sec. On reprend par 200 ml d'éther, lave à l'eau, décante et sèche l'éther. Après évaporation du solvant, on prépare le fumarate à partir de l'huile brute, au sein de l'éthanol. On recueille 7 g de cristaux de fumarate de N-éthyl N-(bis allylamino-4,6 s.triazinyl-2) N'-éthyl N'-(benzothiényl-2 méthyl) éthylène diamine, fondant (Kofler) à 148°C.

L'amide de départ a été préparée à partir du chlorure de l'acide benzothiényl-2 carboxylique et du difumarate de N-(bis allylamino-4,6 s.triazinyl-2) N,N'-diéthyl éthylène diamine, P.F. (Kofler): 204°C, dans le tétrahydrofuranne en présence de triéthylamine.

*Exemples 16 à 19*

Les dérivés suivants ont été préparés selon la méthode décrite dans l'exemple 15:

16) N-éthyl N-(bis allylamino-4,6 s.triazinyl-2) N'-éthyl N'-(p.fluorobenzyl) éthylène diamine, P.F. (Kofler) du difumarate correspondant: 204°C (éthanol).

17) N-éthyl N-(bis allylamino-4,6 s.triazinyl-2) N'-éthyl N'-(benzofurannyl-2 méthyl) éthylène diamine, P.F. (Kofler) du difumarate correspondant: 162°C (éthanol).

18) N-éthyl N-(bis allylamino-4,6 s.triazinyl-2) N'-éthyl N'-cinnamyl éthylène diamine, P.F. (Kofler) du dioxalate correspondant: 171-173°C.

19) N-éthyl N-(bis allylamino-4,6 s.triazinyl-2) N'-éthyl N'-(bis p.fluorophényl-3,3 allyl) éthylène diamine.

**Revendications pour les Etats contractants:**
BE, CH, DE, FR, GB, IT, LI, LU, NL, SE

1. Les polyméthylène diamines N,N'-substituées de formule générale I:

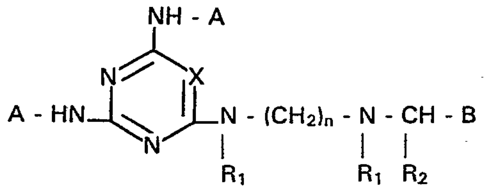

(I)

dans laquelle:

A représente un radical hydrocarboné ayant de 3 à 5 atomes de carbone en chaîne droite ou ramifiée, renfermant éventuellement une ou deux double liaisons, et éventuellement substitué par un ou plusieurs radicaux hydroxyles;

X représente le groupe -CH- ou un atome d'azote;

n représente un nombre entier de 2 à 6;

$R_1$ représente un atome d'hydrogène ou un radical alcoyle ayant de 1 à 5 atomes de carbone;

$R_2$ représente un atome d'hydrogène, un radical alcoyle ayant de 1 à 5 atomes de carbone, un radical cycloalcoyle ayant de 5 à 7 atomes de carbone, ou un radical phényle éventuellement substitué par un ou plusieurs atomes de fluor ou de chlore: et

B représente:

un radical phényle, naphtyle, benzofurannyle, benzothiényle, benzodioxolyle, benzodioxannyle, benzodioxinnyle, Δ3-chroményle, thiochroményle ou chromannyle, chacun éventuellement substitué par un ou plusieurs atomes de fluor ou de chlore, ou radicaux alcoyle ou alcoxy ayant chacun de 1 à 5 atomes de carbone, ou méthylène dioxy; ou

un radical de formule 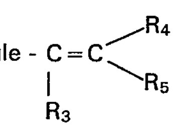

dans laquelle:

$R_3$, $R_4$ et $R_5$ identiques ou différents, représentent chacun un atome d'hydrogène ou un radical phényle éventuellement substitué par un ou plusieurs atomes de chlore ou de fluor.

2. Les sels des composés de la revendication 1 avec des acides appropriés.

3. Les sels selon la revendication 2 qui sont physiologiquement tolérables.

4. La N-éthyl N-(bis allylamino-4,6 s.triazinyl-2) N'-éthyl N'-(bis p.fluorobenzhydryl) éthylène diamine.

5. La N-éthyl N-(bis allylamino-4,6 s.triazinyl-2) N'-éthyl N'-(bis p.fluorobenzhydryl) triméthylène diamine, et son difumarate.

6. La N-éthyl N-(bis allylamino-4,6 s.triazinyl-2) N'-éthyl N'-(benzothiényl-2 méthyl) éthylène diamine, et son fumarate.

7. Un procédé de préparation des composés de la revendication 1 caractérisé en ce que l'on condense une diamine de formule générale II:

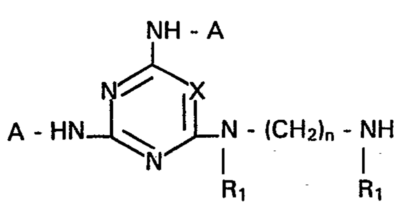

(II)

dans laquelle A, X, n et $R_1$ ont les definitions énoncées dans la revendication 1,

avec un dérivé halogéné de formule générale III:

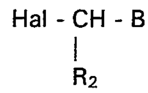

(III)

dans laquelle $R_2$ et B ont les significations définies dans la revendication 1 et Hal représente un atome d'halogène tel que chlore ou brome.

8. Un procédé de préparation des composés de la revendication 1, caractérisé en ce que l'on condense un dérivé halogéné de formule générale IV:

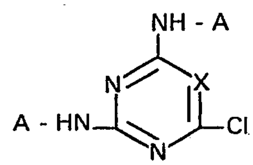

(IV)

dans laquelle A et X ont les significations définies dans la revendication 1,

avec une diamine de formule générale V:

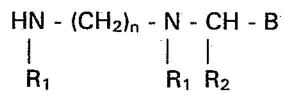

(V)

dans laquelle $R_1$, $R_2$, n et B ont les significations énoncées dans la revendication 1.

9. Un procédé de préparation des composés de la revendication 1, répondant à la formule générale I':

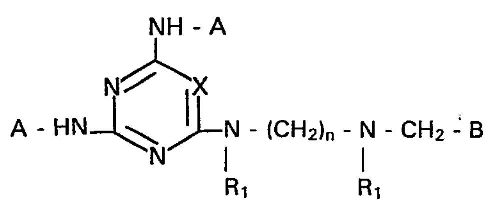

(I')

dans laquelle A, X, n, $R_1$ et B ont les significations définies dans la revendication 1,

caractérisé en ce que l'on réduit l'amide correspondante de formule générale VI:

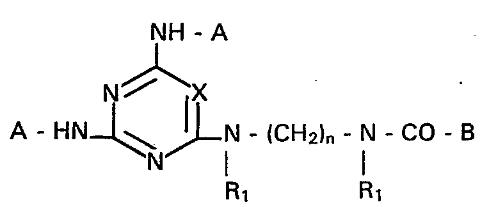

(VI)

dans laquelle A, X, n, $R_1$ et B ont les significations énoncées dans la revendication 1.

10. Les compositions pharmaceutiques contenant comme principe actif un composé selon les revendications 1 ou 3 à 5 avec les excipients pharmaceutiques appropriés.

11. Les compositions pharmaceutiques selon la revendication 9 présentées sous une forme convenant notamment pour le traitement de tout type d'hypoxie tissulaire.

**Revendications pour l'Etat contractant:** AT

1. Procédé de préparation des polyméthylène diamines N, N'-substituées de formule générale I:

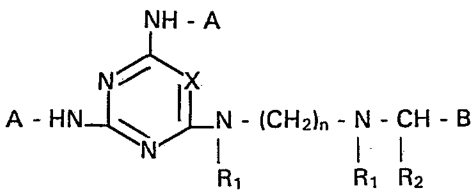

(I)

dans laquelle:

A représente un radical hydrocarboné ayant de 3 à 5 atomes de carbone en chaîne droite ou ramifiée, renfermant éventuellement une ou deux double liaisons, et éventuellement substitué par un ou plusieurs radicaux hydroxyles;

X représente le groupe -CH- ou un atome d'azote;

n représente un nombre entier de 2 à 6;

$R_1$ représente un atome d'hydrogène ou un radical alcoyle ayant de 1 à 5 atomes de carbone;

$R_2$ représente un atome d'hydrogène, un radical alcoyle ayant de 1 à 5 atomes de carbone, un radical cycloalcoyle ayant de 5 à 7 atomes de carbone, ou un radical phényle éventuellement substitué par un ou plusieurs atomes de fluor ou de chlore: et

B représente:

un radical phényle, naphtyle, benzofurannyle, benzothiényle, benzodioxolyle, benzodioxannyle, benzodioxinnyle, Δ3-chroményle, thiochroményle ou chromannyle, chacun éventuellement substitué par un ou plusieurs atomes de fluor ou de chlore, ou radicaux alcoyle ou alcoxy ayant chacun de 1 à 5 atomes de carbone, ou méthylène dioxy; ou

un radical de formule 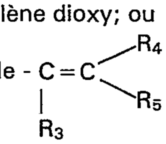

dans laquelle:

$R_3$, $R_4$ et $R_5$ identiques ou différents, représentent chacun un atome d'hydrogène ou un radical phényle éventuellement substitué par un ou plusieurs atomes de chlore ou de fluor;

— et de leurs sels d'additions avec des acides appropriés;
— caractérisé en ce que l'on condense:
— une diamine de formule générale II:

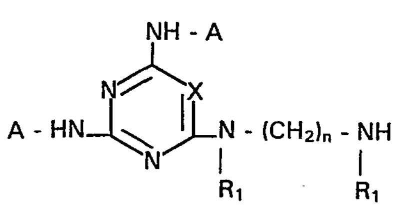

II

dans laquelle A, X, n et $R_1$ ont les definitions énoncées précédemment;

— avec un dérivé halogéné de formule générale III:

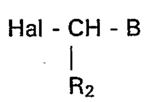

(III)

dans laquelle $R_2$ et B ont les significations définies précédemment et Hal représente un atome d'halogène tel que chlore ou brome;

— et si on le désire on traite les dérivés I ainsi obtenus avec des acides appropriés pour donner les sels d'addition correspondants.

2. Procédé de préparation des polyméthylène diamines N, N'-substitués de formule générale I définie dans la revendication 1

— caractérisé en ce que l'on condense
— un dérivé halogéné de formule générale IV:

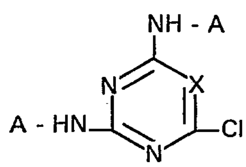

dans laquelle A et X ont les significations définies dans la revendication 1,

— avec une diamine de formule générale V:

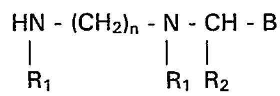

V

dans laquelle $R_1$, $R_2$, n et B ont les significations énoncées dans la revendication 1;

— et si on le désire on traite les dérivés I ainsi obtenus avec des acides appropriés pour donner les sels d'addition correspondants.

3. Procédé de préparation des polyméthylène diamines N, N'-substituées de formule générale I'

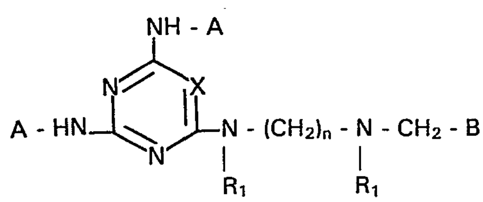

I'

dans laquelle A, X, n, $R_1$ et B ont les significations définies dans la revendication 1,

— caractérisé en ce que l'on réduit l'amide correspondante de formule générale VI:

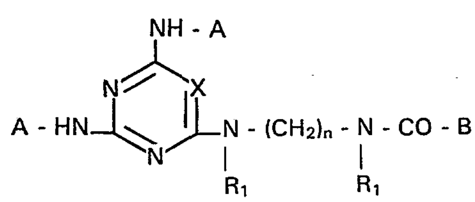

VI

dans laquelle A, X, n, $R_1$ et B ont les significations énoncées dans la revendication 1;

— et si on le désire on traite les dérivés I ainsi obtenus avec des acides appropriés pour donner les sels d'addition correspondants.

**Patentansprüche für die Vertragsstaaten:**
BE, CH, DE, FR, GB, IT, LI, LU, NL, SE

1. N,N'-substituierte Polymethylendiamine der allgemeinen Formel I

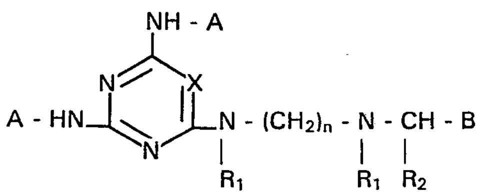
(I)

in der

A eine geradkettige oder verzweigte Kohlenwasserstoffgruppe mit 3 bis 5 Kohlenstoffatomen, die gegebenenfalls eine oder zwei Doppelbindungen aufweist und gegebenenfalls durch eine oder mehrere Hydroxylgruppen substituiert ist;

X eine -CH-Gruppe oder ein Stickstoffatom;

n eine ganze Zahl mit einem Wert von 2 bis 6;

$R_1$ ein Wasserstoffatom oder eine Alkylgruppe mit 1 bis 5 Kohlenstoffatomen;

$R_2$ ein Wasserstoffatom, eine Alkylgruppe mit 1 bis 5 Kohlenstoffatomen, eine Cycloalkylgruppe mit 5 bis 7 Kohlenstoffatomen oder eine gegebenenfalls durch eines oder mehrere Fluoratome oder Chloratome substituierte Phenylgruppe; und

B eine Phenylgruppe, eine Naphthylgruppe, eine Benzofuranylgruppe, eine Benzothienylgruppe, eine Benzodioxolylgruppe, eine Benzodioxanylgruppe, eine Benzodioxinylgruppe, eine $\Delta^3$-Chromenylgruppe, Thiochromenylgruppe oder Chromanylgruppe, die gegebenenfalls durch eines oder mehrere Fluor- oder Chloratome, Alkylgruppen oder Alkoxygruppen mit jeweils 1 bis 5 Kohlenstoffatomen oder Methylendioxygruppen substituiert sein können, oder eine

Gruppe der Formel 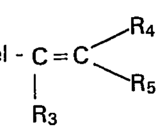

in der

$R_3$, $R_4$ und $R_5$, die gleichartig oder verschieden sein können, jeweils für ein Wasserstoffatom oder eine gegebenenfalls durch eines oder mehrere Chloratome oder Fluoratome substituierte Phenylgruppe stehen,

bedeuten.

2. Die Salze der Verbindungen nach Anspruch 1 mit geeigneten Säuren.

3. Die Salze gemäss Anspruch 2, die physiologisch verträglich sind.

4. N-Ethyl-N-(4,6-bisallylamino-s-triazin-2-yl)-N'-ethyl-N'-(bis-p-fluorbenzhydryl)-ethylendiamin.

5. N-Ethyl-N-(4,6-bisallylamino-s-triazin-2-yl)-N'-ethyl-N'-(bis-p-fluorbenzhydryl)-trimethylendiamin und dessen Difumarat.

6. N-Ethyl-N-(4,6-bisallylamino-s-triazin-2-yl)-N'-ethyl-N'-(benzothien-2-yl-methyl)-ethylendiamin und dessen Fumarat.

7. Verfahren zur Herstellung der Verbindungen nach Anspruch 1, dadurch gekennzeichnet, dass man ein Diamin der allgemeinen Formel II

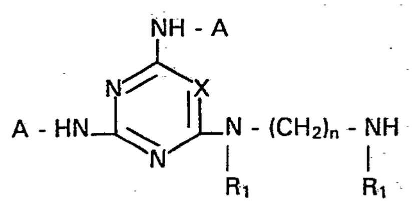
(II)

in der A, X, n und $R_1$ die in Anspruch 1 angegebenen Bedeutungen besitzen,
mit einem Halogenderivat der allgemeinen Formel III

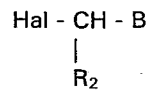
(III)

in der $R_2$ und B die in Anspruch 1 angegebenen Bedeutungen besitzen und Hal für ein Halogenatom, wie ein Chloratom oder ein Bromatom, steht, kondensiert.

8. Verfahren zur Herstellung der Verbindungen nach Anspruch 1, dadurch gekennzeichnet, dass man ein Halogenderivat der allgemeinen Formel IV

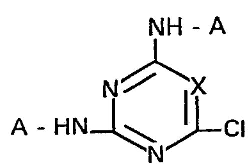
(IV)

in der A und X die in Anspruch 1 angegebenen Bedeutungen besitzen,
mit einem Diamin der allgemeinen Formel V

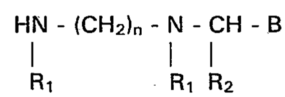
(V)

in der $R_1$, $R_2$, n und B die in Anspruch 1 angegebenen Bedeutungen besitzen, kondensiert.

9. Verfahren zur Herstellung der Verbindungen nach Anspruch 1 der allgemeinen Formel I'

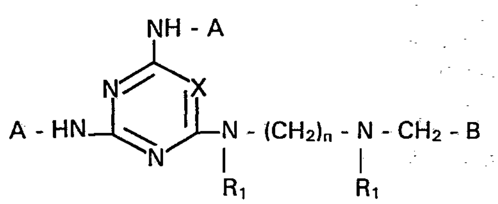
(I')

in der A, X, n, $R_1$ und B die in Anspruch 1 angegebenen Bedeutungen besitzen, dadurch gekennzeichnet, dass man das entsprechende Amid der allgemeinen Formel VI

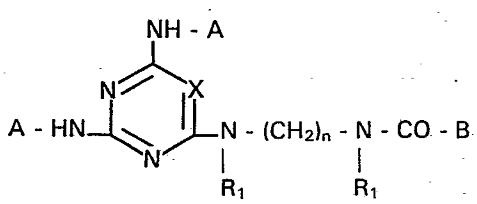
(VI)

in der A, X, n, $R_1$ und B die in Anspruch 1 angegebenen Bedeutungen besitzen, reduziert.

10. Pharmazeutische Zubereitungen enthaltend als Wirkstoff eine Verbindung nach einem der Ansprüche 1 oder 3 bis 5 mit geeigneten pharmazeutischen Trägermaterialien.

11. Pharmazeutische Zubereitungen nach Anspruch 9, dadurch gekennzeichnet, dass sie in einer insbesondere für die Behandlung jeglicher Art von Gewebehypoxie geeigneten Form vorliegen.

**Patentansprüche für den Vertragsstaat:** AT

1. Verfahren zur Herstellung von N,N'-substituierten Polymethylendiaminen der allgemeinen Formel I

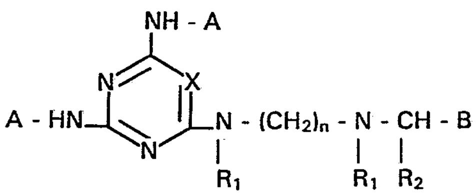

(I)

in der

A eine geradkettige oder verzweigte Kohlenwasserstoffgruppe mit 3 bis 5 Kohlenstoffatomen, die gegebenenfalls eine oder zwei Doppelbindungen aufweist und gegebenenfalls durch eine oder mehrere Hydroxylgruppen substituiert ist;

X eine -CH-Gruppe oder ein Stickstoffatom;

n eine ganze Zahl mit einem Wert von 2 bis 6;

$R_1$ ein Wasserstoffatom oder eine Alkylgruppe mit 1 bis 5 Kohlenstoffatomen;

$R_2$ ein Wasserstoffatom, eine Alkylgruppe mit 1 bis 5 Kohlenstoffatomen, eine Cycloalkylgruppe mit 5 bis 7 Kohlenstoffatomen oder eine gegebenenfalls durch eine oder mehrere Fluoratome oder Chloratome substituierte Phenylgruppe; und

B eine Phenylgruppe, eine Naphthylgruppe, eine Benzofuranylgruppe, eine Benzothienylgruppe, eine Benzodioxolylgruppe, eine Benzodioxanylgruppe, eine Benzodioxinylgruppe, eine $\Delta^3$-Chromenylgruppe, Thiochromenylgruppe oder Chromanylgruppe, die gegebenenfalls durch eines oder mehrere Fluor- oder Chloratome, Alkylgruppen oder Alkoxygruppen mit jeweils 1 bis 5 Kohlenstoffatomen oder Methylendioxygruppen substituiert sein können, oder eine

Gruppe der Formel 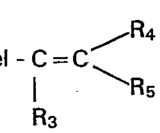

in der

$R_3$, $R_4$ und $R_5$, die gleichartig oder verschieden sein können, jeweils für ein Wasserstoffatom oder eine gegebenenfalls durch eines oder mehrere Chloratome oder Fluoratome substituierte Phenylgruppe stehen,

bedeuten, und ihrer Säureadditionssalze mit geeigneten säuren, dadurch gekennzeichnet, dass man ein Diamin der allgemeinen Formel II

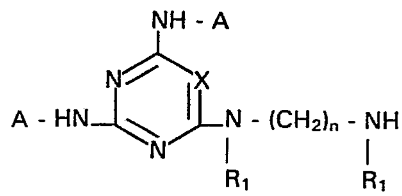

(II)

in der A, X, n und $R_1$ die oben angegebenen Bedeutungen besitzen,

mit einem Halogenderivat der allgemeinen Formel III

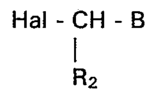

(III)

in der $R_2$ und B die oben angegebenen Bedeutungen besitzen und Hal für ein Halogenatom, wie ein Chloratom oder ein Bromatom, steht, kondensiert;

und gewünschtenfalls die in dieser Weise erhaltenen Derivate I mit geeigneten Säuren zur Bildung der entsprechenden Additionssalze behandelt.

2. Verfahren zur Herstellung der N,N'-substituierten Polymethylendiamine der in Anspruch 1 definierten allgemeinen Formel I, dadurch gekennzeichnet, dass man ein Halogenderivat der allgemeinen Formel IV

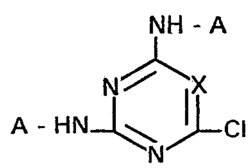

(IV)

in der A und X die in Anspruch 1 angegebenen Bedeutungen besitzen,

mit einem Diamin der allgemeinen Formel V

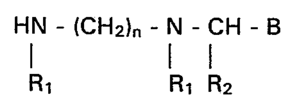

(V)

in der $R_1$, $R_2$, n und B die in Anspruch 1 angegebenen Bedeutungen besitzen, kondensiert;

und gewünschtenfalls die in dieser Weise erhaltenen Derivate I mit geeigneten Säuren zur Bildung der entsprechenden Additionssalze behandelt.

3. Verfahren zur Herstellung von N,N'-substituierten Polymethylendiaminen der allgemeinen Formel I'

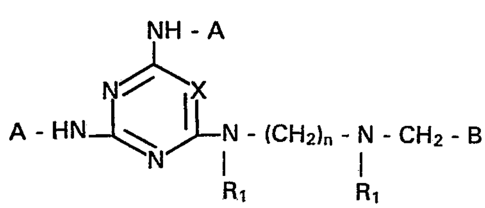

(I')

in der A, X, n, $R_1$ und B die in Anspruch 1 angegebenen Bedeutungen besitzen, dadurch gekennzeichnet, dass man das entsprechende Amid der allgemeinen Formel VI

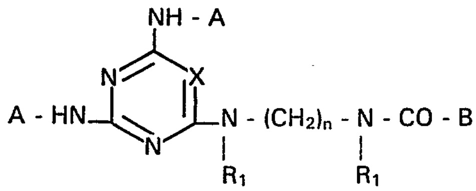
(VI)

in der A, X, n, $R_1$ und B die in Anspruch 1 angegebenen Bedeutungen besitzen, reduziert;
und gewünschtenfalls die in dieser Weise erhaltenen Derivate I mit geeigneten Säuren zur Bildung der entsprechenden Additionssalze behandelt.

**Claims for the Contracting States:**
BE, CH, DE, FR, GB, IT, LI, LU, NL, SE

1. The N,N'-substituted polymethylenediamines of the general formula I

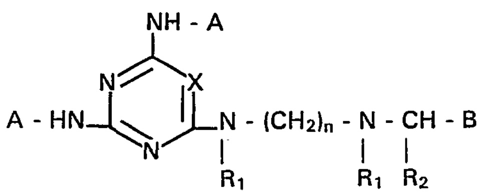
(I)

in which

A represents a straight-chain or branched hydrocarbon radical having from 3 to 5 carbon atoms, optionally containing one or two double bonds and optionally substituted by one or more hydroxy radicals;

X represents the group -CH- or a nitrogen atom;

n represents an integer from 2 to 6;

$R_1$ represents a hydrogen atom or an alkyl radical having from 1 to 5 carbon atoms;

$R_2$ represents a hydrogen atom, an alkyl radical having from 1 to 5 carbon atoms, a cycloalkyl radical having from 5 to 7 carbon atoms, or a phenyl radical optionally substituted by one or more fluorine or chlorine atoms; and

B represents a phenyl, naphthyl, benzofuranyl, benzothienyl, benzodioxolyl, benzodioxanyl, benzodioxinyl, $\Delta^3$-chromenyl, thiochromenyl or chromanyl radical each optionally substituted by one or more fluorine or chlorine atoms or alkyl or alkoxy radicals each having from 1 to 5 carbon atoms or methylenedioxy; or a radical of the formula

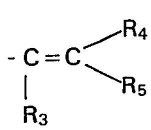

in which:

$R_3$, $R_4$ and $R_5$ are the same or different and each represents a hydrogen atom or a phenyl radical optionally substituted by one or more chlorine or fluorine atoms.

2. The salts of the compounds of claim 1 with suitable acids.

3. The salts according to claim 2 that are physiologically acceptable.

4. N-ethyl-N-(4,6-bis-allylamino-s.-triazin-2-yl)-N'-ethyl-N'-(bis-p-fluorobenzhydryl)-ethylenediamine.

5. N-ethyl-N-(4,6-bis-allylamino-s.-triazin-2-yl)-N'-ethyl-N'-(bis-p-fluorobenzhydryl)-trimethylenediamine and its difumarate.

6. N-ethyl-N-(4,6-bis-allylamino-s.-triazin-2-yl)-N'-ethyl-N'-(2-benzothienylmethyl)-ethylenediamine and its fumarate.

7. A process for the preparation of the compounds of claim 1, characterised in that a diamine of the general formula II:

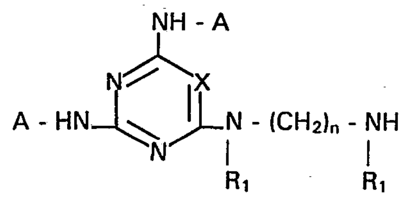
II

in which A, X, n and $R_1$ have the meanings given in claim 1 is condensed with a halogen derivative of the general formula III:

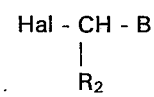
(III)

in which $R_2$ and B have the meanings defined in claim 1 and Hal represents a halogen atom, such as chlorine or bromine.

8. A process for the preparation of the compounds of claim 1, characterised in that a halogen derivative of the general formula IV:

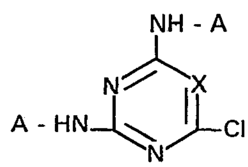
(IV)

in which A and X have the meanings defined in claim 1 is condensed with a diamine of the general formula V:

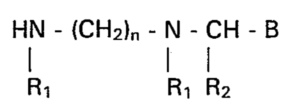
(V)

in which $R_1$, $R_2$, n and B have the meanings given in claim 1.

9. A process for the preparation of the compounds of claim 1, corresponding to the general formula I':

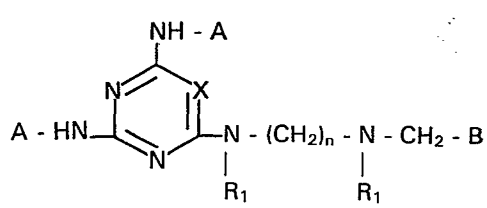
(I')

in which A, X, n, $R_1$ and B have the meanings defined in claim 1,
characterised in that the corresponding amide of the general formula VI:

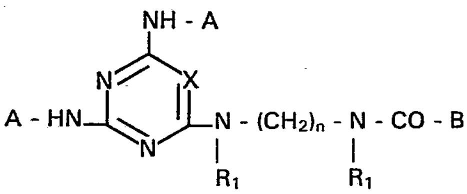

(VI)

in which A, X, n, $R_1$ and B have the meanings given in claim 1 is reduced.

10. Pharmaceutical compositions containing as active ingredient a compound according to claims 1 or 3 to 5 with suitable pharmaceutical excipients.

11. Pharmaceutical compositions according to claim 9 presented in a form suitable particularly for the treatment of all types of tissue hypoxia.

**Claims for the Contracting State:** AT

1. Process for the preparation of the N,N'-substituted polymethylenediamines of the general formula I:

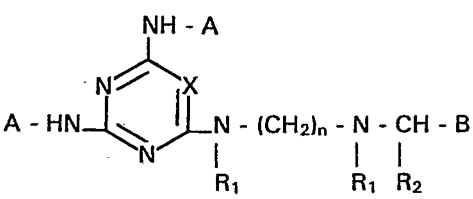

(I)

in which

A represents a straight-chain or branched hydrocarbon radical having from 3 to 5 carbon atoms, optionally containing one or two double bonds and optionally substituted by one or more hydroxy radicals;

X represents the group -CH- or a nitrogen atom;

n represents an integer from 2 to 6;

$R_1$ represents a hydrogen atom or an alkyl radical having from 1 to 5 carbon atoms;

$R_2$ represents a hydrogen atom, an alkyl radical having from 1 to 5 carbon atoms, a cycloalkyl radical having from 5 to 7 carbon atoms, or a phenyl radical optionally substituted by one or more fluorine or chlorine atoms; and

B represents a phenyl, naphthyl, benzofuranyl, benzothienyl, benzodioxolyl, benzodioxanyl, benzodioxinyl, $\Delta^3$-chromenyl, thiochromenyl or chromanyl radical each optionally substituted by one or more fluorine or chlorine atoms or alkyl or alkoxy radicals each having from 1 to 5 carbon atoms or methylenedioxy; or a radical of the formula

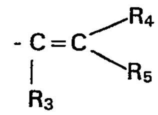

in which:

$R_3$, $R_4$ and $R_5$ are the same or different and each represents a hydrogen atom or a phenyl radical optionally substituted by one or more chlorine or fluorine atoms,

and their addition salts with suitable acids; characterised in that a diamine of the general formula II:

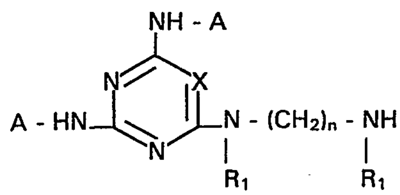

II

in which A, X, n and $R_1$ have the meanings given above is condensed with a halogen derivative of the general formula III:

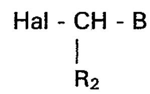

(III)

in which $R_2$ and B have the meanings defined above and Hal represents a halogen atom, such as chlorine or bromine,

and if desired the derivatives I so obtained are treated with suitable acids to give the corresponding addition salts.

2. Process for the preparation of the N,N'-substituted polymethylenediamines of the general formula I defined in claim 1,
characterised in that a halogen derivative of the general formula IV:

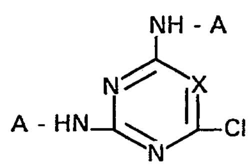

(IV)

in which A and X have the meanings defined in claim 1 is condensed with a diamine of the general formula V:

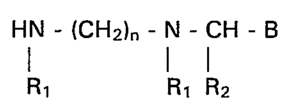

(V)

in which $R_1$, $R_2$, n and B have the meanings given in claim 1;
and if desired the derivatives I so obtained are treated

with suitable acids to give the corresponding addition salts.

3. Process for the preparation of N,N'-substituted polymethylenediamines of the general formula I':

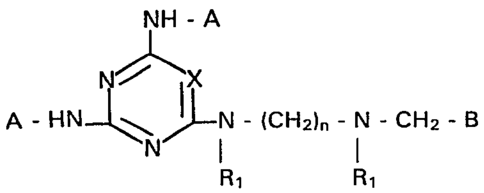

(I')

in which A, X, n, $R_1$ and B have the meanings defined in claim 1,

characterised in that the corresponding amide of the general formula VI:

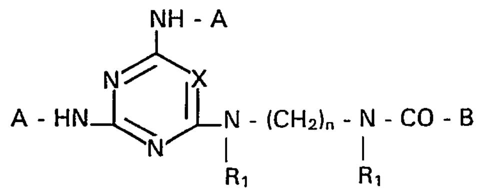

(VI).

in which A, X, n, $R_1$ and B have the meanings given in claim 1 is reduced;
and if desired the derivatives I so obtained are treated with suitable acids to give the corresponding addition salts.